# FASCICULE DE BREVET EUROPEEN

(11) **EP 3 423 072 B1**
(45) Date de publication et mention de la délivrance du brevet: **07.06.2023**
(21) Numéro de dépôt: 17707061.2
(22) Date de dépôt: 01.03.2017
(51) Int. Cl.: A61K 35/747, A61P 15/02, A61K 31/095, A61K 45/06, A61K 31/198, A61K 33/04, A61K 38/06

(54) **COMPOSITIONS POUR LE TRAITEMENT DES CANDIDOSES**
ZUSAMMENSETZUNGEN ZUR BEHANDLUNG VON CANDIDIASIS-INFEKTIONEN
COMPOSITIONS FOR TREATING CANDIDIASIS INFECTIONS

(30) Priorité: 01.03.2016 FR 1651717
(43) Date de publication de la demande: 09.01.2019
(73) Titulaire: NEXBIOME THERAPEUTICS, 63000 Clermont-Ferrand (FR)
(72) Inventeur: NIVOLIEZ, Adrien, 15 000 AURILLAC (FR)
(74) Mandataire: Icosa
(86) Numéro de dépôt international: PCT/EP2017/054701
(87) Numéro de publication internationale: WO 2017/148975

(56) Documents cités:
- WO-A1-98/46261
- WO-A1-2014/009349
- FR-A1- 2 992 973

## Description

### DOMAINE DE L'INVENTION

La présente invention concerne l'utilisation combinée de fortes teneurs en lactobacilles (de 5.10¹⁰ à 1.10¹² UFC) avec 15 à 300 mg de thiosulfate pour le traitement en première intention des candidoses et des candidoses récidivantes. La combinaison selon l'invention peut remplacer le traitement par un antifongique chimique.

L'invention est par exemple adaptée pour le traitement en première intention des candidoses vaginales et des candidoses vaginales récidivantes.

### ETAT DE LA TECHNIQUE

Chez une femme saine, la flore urogénitale comprend près de 50 espèces différentes de microorganismes. Parmi ces microorganismes, 95 % de la population est constituée de diverses souches de lactobacilles, aussi appelées « bacilles de Döderlein ». Ces lactobacilles jouent un rôle de protection contre les pathogènes par divers mécanismes : production de peroxyde d'hydrogène, production d'acide lactique, production de bactériocines, inhibition de l'adhésion et de l'expansion des pathogènes. En particulier, ces lactobacilles maintiennent un pH acide par la production d'acide lactique à partir du glycogène présent dans le mucus vaginal. Ainsi, la croissance de nombreux pathogènes du microbiote vaginal tels que *Gardnerella vaginalis, Prevotella bivia, Neisseria gonorrhoeae, Mycoplasma,* et *Mobiluncus* est inhibée.

Le microbiote vaginal normal est ainsi principalement composé de lactobacilles formant un biofilm protecteur à la surface de la muqueuse. Les lactobacilles les plus fréquemment identifiés dans le vagin sont notamment *Lactobacillus crispatus, Lactobacillus jensenii, Lactobacillus vaginalis, Lactobacillus iners* et *Lactobacillus gasseri.*

La candidose vulvovaginale (CVV) est une infection mycosique liée à un excès de croissance de champignons à partir d'un microbiote normal. 85-95 % des CVV sont liées à *Candida albicans.* La candidose vulvovaginale touche 70-75 % des femmes en période d'activité génitale au moins une fois dans leur vie (1) ; environ 40-50 % d'entre elles présenteront un deuxième épisode. L'incidence de la candidose vulvovaginale récidivante (définie par la survenue d'au moins 4 épisodes par an, dont deux prouvés par l'examen mycologique) a été estimée à 5-8 %. Cette affection bénigne a un impact très négatif sur la qualité de vie des patientes et engendre des dépenses de santé importantes. Traiter une telle pathologie est difficile du fait de la pathogénèse multifactorielle de cette affection.

L'apparition d'une CVV et sa propension à récidiver sont un réel problème de santé publique et représente des coûts de santé importants. Les traitements actuels contre les CVV, basés sur l'utilisation d'antifongiques, sont souvent associés à des effets secondaires tels que le développement de vaginoses bactériennes et induisent de nombreuses récidives (2).

Les nouveaux développements thérapeutiques doivent s'orienter vers une prise en compte plus large de la pathologie en tenant compte de son environnement spécifique et des causes de son apparition. Ainsi, depuis quelques années, les caractéristiques du microbiote vaginal sont particulièrement étudiées. Ce microbiote est dominé par le genre *Lactobacillus,* présent chez environ 70 % des femmes dont chaque espèce individuelle constitue 99 % des ribotypes observés chez une femme (3). Des produits probiotiques ont été développés et testés pour prévenir les récidives aux CVV (4, 5, 6) via le rétablissement de l'équilibre du microbiote vaginal. L'action de ces produits ne se situe pas directement contre le pathogène responsable de la pathologie mais sur la prévention d'une nouvelle dysbiose pouvant être induite par le traitement antifongique lui-même ou par d'autres éléments extérieurs.

L'administration de lactobacilles 'bénéfiques' par voie orale ou vaginale a été décrite pour promouvoir la santé vaginale. En particulier, les demandes de brevet WO 84/04675, WO 2000/035465, US 2002/0044926 et WO 2006/045475 décrivent l'administration par voie orale ou vaginale de bactéries lactiques pour promouvoir la santé vaginale et pour prévenir les récidives de la candidose vulvovaginale.

Les lactobacilles préférés sont les *Lactobacillus rhamnosus, Lactobacillus crispatus* et *Lactobacillus vaginalis.* Les brevets US 6,093,394, US 6,468,526 et US 7,807,440, ainsi que la demande de brevet US 2010/0151026, décrivent l'administration de souches spécifiques de *Lactobacillus crispatus.*

Ces lactobacilles peuvent être administrés sous forme lyophilisée ou en solution, et éventuellement en combinaison avec d'autres agents actifs.

Les demandes de brevet WO2014/009349 et WO 2014/009330 décrivent l'utilisation de thiosulfate pour potentialiser l'effet anti-pathogène des lactobacilles. Dans ces demandes, du thiosulfate est ajouté dans le milieu de culture du lactobacille, avant lyophilisation, à une concentration optimale de 113 g/L pour environ 10⁸ UFC/ml de lactobacilles (formulation A). Cet ajout permet de potentialiser l'effet anti-pathogène des lactobacilles, suite à une étape de culture préalable. Dans tous les essais, les inocula sont préparés par le mode opératoire suivant :
- Préparation des inocula :
   ∘ Produit probiotique : on place 0,2 g du lyophilisat dans 20 ml de bouillon MRS à l'étuve à 37°C pendant 48 h ;
   ∘ Souche pathogène : on place 0,2 ml dans 20 ml de bouillon Sabouraud à 25°C pendant 48 h ;
- Mise en contact des inocula : on place 5 ml de la souche pathogène avec 5 ml de la souche probiotique ;
- Suivi : détermination numéraire du pathogène et du probiotique à T0, T4 h, T24 h et T28 h. La numération des souches de lactobacilles reste constante et celle du pathogène diminue.

Ces demandes montrent que l'ajout de thiosulfate dans le milieu de culture avant lyophilisation de la souche de lactobacille) à une concentration allant de 1 g/L à 113 g/L, permet de potentialiser l'effet anti-pathogène de ces lactobacilles, qui ont été préalablement préparés par une pré-culture pendant 48 h avant mise en contact avec le pathogène. Sans cette étape de pré-culture, aucune inhibition n'est observable dans le temps suivi de co-culture.

Ces demandes montrent que l'ajout de thiosulfate permet d'envisager l'utilisation des lactobacilles potentialisés pour recoloniser le microbiote vaginal et empêcher la repousse des agents pathogènes et ainsi les récidives. Elles ne permettent pas d'envisager une utilisation des lactobacilles potentialisés, formulés dans une composition pharmaceutique, en traitement de première intention des CVV, au vu du besoin de pré-culture de la souche avant utilisation qui traduit une nonadaptation de la composition à agir immédiatement sur le pathogène (et ainsi à exercer une action curative comme l'exercerait un antifongique) mais au contraire une adaptation de la composition pour exercer un effet de barrière à une recolonisation par le pathogène après un traitement antifongique.

Dans ces demandes, les concentrations en lactobacilles indiquées de manière générale vont de 10⁷ à 10¹⁰ UFC/g de composition, la concentration de 10⁸ UFC/ml avant lyophilisation étant la concentration préférée employée dans les exemples (formulation A).

Malgré cette utilisation dans la prévention des récidives aux CVV, l'utilisation de souches de lactobacilles en première intention en substitution des antifongiques est non admise, notamment au vu que la proportion des lactobacilles ne varie pas au cours du développement du pathogène et de la pathologie.

De manière surprenante, il a été constaté qu'une composition pharmaceutique comprenant une concentration spécifique en lactobacilles et en thiosulfate pouvait être utilisée en traitement de première intention des candidoses et des candidoses récidivantes. L'invention décrit en particulier les candidoses vulvovaginales et les candidoses vulvovaginales récidivantes.

De plus, les traitements actuels chimiques (antibiotiques et antifongiques) ne respectent pas l'équilibre du microbiote et peuvent favoriser l'émergence d'effets secondaires tels que le développement de vaginoses bactériennes et de nombreuses récidives (2). Cette invention permettra par sa composition de prévenir les risques de récidives. L'invention développée permet donc par l'utilisation d'une souche probiotique d'inhiber spécifiquement des pathogènes responsables de ces pathologies, tout en respectant le microbiote lactobacillaire caractéristique de la femme asymptomatique (2). L'utilisation d'un produit biologique naturel, à l'inverse de produits chimiques, ne génère pas de dysbiose imputable aux traitements actuels et favorise ainsi la prévention des récidives.

### EXPOSE DE L'INVENTION

L'invention porte sur l'utilisation, en tant que traitement de première intention des candidoses et des candidoses récidivantes, d'une concentration spécifique en lactobacilles (concentration de 5.10¹⁰ à 1.10¹² UFC par prise), hors des utilisations usuelles de ces produits, associée à 15 à 300 mg de thiosulfate. Cette invention nous permet de proposer un traitement en première intention des candidoses avec une posologie équivalente aux antifongiques actuels.

### DESCRIPTION DETAILLEE DE L'INVENTION

La présente invention a pour objet une composition pharmaceutique comprenant i) à titre d'actif thérapeutique de 5.10¹⁰ à 1.10¹² UFC de lactobacilles et ii) de 15 à 300 mg de thiosulfate en tant que produits de combinaison pour son utilisation en tant que traitement de première intention des candidoses et des candidoses récidivantes. L'invention décrit en particulier les candidoses vulvovaginales et les candidoses vulvovaginales récidivantes.

Le terme « candidose » désigne une infection fongique causée par des levures du genre *Candida. Candida albicans,* l'espèce la plus fréquente, fait partie de la flore habituelle de l'oro-pharynx, du tube digestif, et peut aussi être présent en faible quantité dans le microbiote vaginal normal. La candidose vulvovaginale est très fréquente et peut être sujette à plusieurs récidives.

Par « traitement de première intention des candidoses et des candidoses récidivantes », on entend un traitement thérapeutique, avantageusement curatif, présentant des propriétés fongicides permettant de traiter ces candidoses, et ainsi concrètement une destruction des pathogènes à l'origine de l'infection. Dans le cas présent, le traitement présente également des propriétés fongistatiques, caractérisées par l'inhibition de la croissance desdits pathogènes.

Selon l'invention, les pathogènes appartiennent au genre *Candida,* et préférentiellement aux espèces de *Candida albicans, Candida glabrata, Candida tropicalis,* tout préférentiellement à l'espèce des *Candida albicans. Candida albicans* est l'espèce de levure la plus fréquente et la plus connue du genre *Candida.* Elle provoque des infections fongiques (candidose), par exemple au niveau des muqueuses gynécologiques.

A titre de candidoses, on peut citer :
- Les candidoses cutanéomuqueuses, en particulier
   o Les candidoses au niveau bucco-digestif : les candidoses buccales (muguet, glossite, stomatite, langue noire villeuse), les candidoses digestives (oesophagite, gastro-entérite, colite, anite),
   o les candidoses génitales : vulvite et vulvo-vaginite, balanite et méatite
   o les candidoses cutanées : intertrigo des grands plis (plis inguinaux, pli interfessier, plis sous-mammaires et replis abdominaux, creux axillaires, dermite fessière des nourrissons), intertrigo des petits plis (espaces interdigitaux des mains)
   o les candidoses des ongles : onyxis, onycholyse
- Les candidoses oesophagiennes
- Les candidoses systémiques

Le terme « lactobacilles » désigne l'ensemble des bactéries du genre *Lactobacillus,* bactéries à Gram positif, immobiles, de formes et dimensions variables, et aéro-anaérobies facultatives. La plupart des lactobacilles convertissent le lactose et d'autres sucres simples en acide lactique. Dans l'exemple du traitement des CVV, les lactobacilles colonisent le vagin et constituent un élément important du microbiote vaginale. La présente invention peut ainsi être relative aux lactobacilles constituant le microbiote vaginal, en particulier *Lactobacillus casei,* et *Lactobacillus rhamnosus.*

Le terme « UFC » désigne l'unité de mesure généralement reconnue par l'homme du métier pour quantifier les bactéries capables de fonder une colonie et signifie précisément « Unité Formant Colonie ». Il est exprimé par rapport au poids total de la composition pharmaceutique.

Le terme « composition pharmaceutique » désigne le traitement thérapeutique administré au patient. Dans la généralité, la « composition pharmaceutique » correspondra à une dose du médicament. Toutefois, ne sortiraient pas du cadre de l'invention, des unités distinctes prises au même moment et qui de manière cumulée contiendraient les produits de l'invention dans les teneurs revendiquées. Dans le cadre de la présente description, les teneurs sont données pour une composition correspondant à une dose, et pourront être adaptées dans les autres cas.

Le terme « produits de combinaison » signifie que les lactobacilles et le thiosulfate sont formulés soit dans une même composition, soit dans deux compositions distinctes administrées au patient de manière concomitante, c'est-à-dire au même moment dans la journée.

Tout intervalle de valeurs désigné par l'expression « entre a et b » représente le domaine de valeurs allant de plus de a à moins de b (c'est-à-dire bornes a et b exclues) tandis que tout intervalle de valeurs désigné par l'expression « de a à b » signifie le domaine de valeurs allant de a à b (c'est-à-dire incluant les bornes strictes a et b).

Sauf indication contraire, tous les pourcentages sont des pourcentages en poids.

D'une manière surprenante, il a été découvert que lorsque ces lactobacilles sont utilisés à une concentration de 5.10¹⁰ à 1.10¹² UFC, en présence de 15 à 300 mg de thiosulfate dans une composition pharmaceutique, cette composition avait la capacité d'inhiber rapidement et spécifiquement des levures du genre *Candida.* Ainsi, contrairement à toute attente, à cette concentration, les lactobacilles, potentialisés par le thiosulfate, peuvent être utilisés à titre d'actif thérapeutique ayant une action antifongique. Concrètement, il est possible de proposer, en lieu et place d'un antifongique chimique, les compositions selon l'invention dans la prise en charge du traitement des candidoses, telles que les candidoses vulvovaginales et les candidoses vulvovaginales récidivantes. Le traitement peut même consister en la seule administration des compositions selon l'invention, sans administration, y compris préalable, d'un antifongique chimique tel que le fluconazole ou le ketoconazole. Les antifongiques chimiques conduisent à un déséquilibre du microbiote par exemple. Les compositions selon l'invention peuvent donc remplacer les antifongiques habituellement administrés dans la prise en charge de ces conditions. L'invention a donc également pour objet une composition selon l'invention pour son utilisation en substitution à un antifongique chimique dans le traitement des candidoses, telles que les candidoses vulvovaginales et les candidoses vulvovaginales récidivantes.

La composition selon l'invention peut être utilisée pour inhiber, en moins de 24 heures et sans pré-culture, les pathogènes du genre *Candida,* en particulier *Candida albicans* ou *Candida glabrata.*

Selon l'invention, l'actif thérapeutique ayant une action antifongique est une association de lactobacilles et de thiosulfate. Cette association pourra prendre la forme d'une seule composition (utilisation simultanée), de deux compositions administrées en même temps (utilisation combinée).

Selon l'invention, le thiosulfate peut être choisi parmi le thiosulfate de sodium ou le thiosulfate de potassium. Préférentiellement, le thiosulfate de sodium est utilisé. Le thiosulfate de sodium est constitué d'ions sodium et d'ions thiosulfate.

La composition comprend de 15 à 300 mg de thiosulfate, avantageusement 30 à 300 mg, plus avantageusement 45 à 120 mg, encore plus avantageusement 45 à 80 mg, et idéalement de 60 mg.

A une teneur inférieure à 15 mg, la quantité de thiosulfate n'est pas suffisante pour potentialiser les lactobacilles. A une teneur supérieure à 300 mg, le thiosulfate commence à exercer une action d'inhibition délétère sur les lactobacilles endogènes ou exogènes.

La composition pharmaceutique comprend de 5.10¹⁰ à 1.10¹² UFC de lactobacilles. La quantité en lactobacilles varie avantageusement de 1.10¹¹ à 1.10¹² UFC.

A une teneur inférieure à 2.10¹⁰ UFC de lactobacilles, on n'observe pas d'effet d'inhibition des pathogènes sans culture préalable de la souche, ce qui ne permet pas d'envisager une action en traitement, en particulier en première intention.

En particulier, pour des teneurs d'environ 5.10¹⁰ UFC de lactobacilles ou plus, on observe un effet d'inhibition dès 24h. Selon un mode particulier de réalisation de l'invention, la composition pharmaceutique comprend environ 5.10¹⁰ UFC de lactobacilles.

Ces teneurs correspondent à la généralité d'une composition correspondant à une dose, et pourront être adaptées dans les autres cas.

La composition est avantageusement préparée à partir d'un lyophilisat comprenant les lactobacilles et le thiosulfate.

Dans une première variante, le thiosulfate est introduit lors de la lyophilisation des lactobacilles.

Avantageusement, la composition comprend de 250 à 2500 mg de lyophilisat, plus avantageusement de 500 à 1500 mg de lyophilisat ; le lyophilisat comprenant les lactobacilles et le thiosulfate.

L'homme du métier, spécialiste de la lyophilisation, saura adapter la quantité de thiosulfate ajoutée avant lyophilisation pour obtenir la quantité désirée dans la poudre finale obtenue après lyophilisation.

Dans une deuxième variante, les lactobacilles sont lyophilisés en absence de thiosulfate, lequel est introduit ultérieurement dans la composition pharmaceutique ou est co-administré avec la composition comprenant le lyophilisat de lactobacilles. Avantageusement, la composition pharmaceutique comprend
- de 250 à 2500 mg de lyophilisat, plus avantageusement de 500 à 1500 mg de lyophilisat ; le lyophilisat comprenant les lactobacilles ;
- de 15 à 300 mg de thiosulfate, plus avantageusement de 30 à 300 mg, encore plus avantageusement 45 à 120 mg, encore plus avantageusement 45 à 80 mg, et idéalement de 60 mg de thiosulfate.

Dans une troisième variante, les première et deuxième variantes sont combinées : une partie du thiosulfate est introduite lors de la lyophilisation, l'autre partie lors de la formulation de la composition pharmaceutique. L'homme du métier saura adapter les proportions dans le respect des teneurs décrites précédemment dans les première et deuxième variantes.

Selon un aspect préféré de l'invention, les lactobacilles sont des *Lactobacillus rhamnosus* et/ou des *Lactobacillus crispatus et*/*ou Lactobacillus casei et*/*ou Lactobacillus vaginalis.* Avantageusement, les lactobacilles sont des *Lactobacillus rhamnosus* et/ou des *Lactobacillus crispatus et*/*ou Lactobacillus casei.* La souche préférée est le *L. rhamnosus* Lcr35^{®}. (7).

De préférence, les lactobacilles sont sous forme lyophilisée. La souche peut être le seul élément lyophilisé de la composition, mais de préférence la souche est lyophilisée dans un milieu comprenant des constituants additionnels, qui seront ajoutés avant ou après l'étape de lyophilisation. Dans une variante le thiosulfate peut être ajouté avant lyophilisation. Dans une autre variante, le thiosulfate est ajouté après lyophilisation lors de la préparation de la composition pharmaceutique.

Selon un autre aspect particulier de l'invention, la composition pharmaceutique comprend de plus une matrice de conservation et/ou des excipients bien connus de l'homme du métier, et optionnellement d'autres principes actifs ayant une action complémentaire.

En particulier, cette composition peut comprendre les principes actifs suivants : des hormones (estriol, progestérone...), des agents anti-inflammatoires. L'homme du métier saura déterminer quels sont les principes actifs qui peuvent être avantageusement couplés aux lactobacilles. Cette composition comprend également, selon un aspect spécifique de l'invention, plusieurs souches de lactobacilles.

Ces compositions pharmaceutiques sont avantageusement formulées pour une administration par voie vaginale, orale ou topique, plus avantageusement vaginale pour les candidoses vulvovaginales. En particulier la galénique utilisée sera sous forme de gélules, de comprimés, de crèmes, de suspensions liquides ou huileuses, ou tout autre dispositif médical approprié. Avantageusement, la galénique utilisée sera sous forme de gélules ou de comprimés. L'homme du métier saura adapter la forme appropriée à l'effet thérapeutique escompté.

La composition pharmaceutique selon l'invention est avantageusement à libération immédiate, en particulier un comprimé ou une gélule à libération immédiate ou modifiée selon l'effet recherché.

Les modes d'administration, les posologies et les formes galéniques optimales des composés et compositions selon l'invention peuvent être déterminés selon les critères généralement pris en compte dans l'établissement d'un traitement pharmaceutique adapté à un patient, comme par exemple l'âge ou le poids corporel du patient, la gravité de son état général, la tolérance au traitement, les effets secondaires constatés. Ce patient pourra être l'Homme ou un animal, avantageusement l'Homme.

L'invention a également pour objet une composition pharmaceutique pour administration par voie vaginale caractérisée en ce qu'elle est sous la forme d'un produit de combinaison comprenant i) une première composition pharmaceutique comprenant à titre d'actif thérapeutique de 5.10¹⁰ à 1.10¹² UFC de lactobacilles, et ii) une deuxième composition pharmaceutique comprenant de 15 à 300 mg de thiosulfate, pour son utilisation séparée, simultanée ou décalée dans le temps en tant que traitement de première intention des candidoses et des candidoses récidivantes.

Les lactobacilles et le thiosulfate sont avantageusement tels que décrits précédemment.

La composition pharmaceutique comprend avantageusement 30 à 300 mg, plus avantageusement 45 à 120 mg, encore plus avantageusement 45 à 80 mg, et idéalement 60 mg de thiosulfate.

Dans la composition pharmaceutique, la teneur en lactobacilles varie avantageusement de 1.10¹¹ à 1.10¹² UFC.

Selon un mode particulier de réalisation de l'invention, la composition comprend environ 5.10¹⁰ UFC de lactobacilles.

La composition pharmaceutique comprend avantageusement :
- de 250 à 2500 mg de lyophilisat, plus avantageusement de 500 à 1500 mg de lyophilisat ; le lyophilisat comprenant les lactobacilles et la composition comprend de 5.10¹⁰ à 1.10¹² UFC de lactobacilles;
- de 15 à 300 mg de thiosulfate, plus avantageusement de 30 à 300 mg, encore plus avantageusement 45 à 120 mg, encore plus avantageusement 45 à 80 mg, et idéalement de 60 mg de thiosulfate

La composition pharmaceutique est avantageusement à libération immédiate, en particulier un comprimé ou une gélule à libération immédiate. Selon un autre mode de réalisation de l'invention, la composition pharmaceutique est avantageusement à libération contrôlée, en particulier un comprimé ou une gélule à libération contrôlée.

Par libération contrôlée, on entend en particulier une libération du la souche de Lactobacille rapide et également progressive sur au moins 24h. On emploie parfois indifféremment libération contrôlée et libération prolongée.

Dans un mode de réalisation particulier, le comprimé à libération immédiate selon l'invention est combiné avec une formulation à libération prolongée comprenant elle aussi des lactobacilles selon l'invention.

L'invention a également pour objet la composition pharmaceutique comprenant i) à titre d'actif thérapeutique de 5.10¹⁰ à 1.10¹² UFC de lactobacilles et ii) de 15 à 300 mg de thiosulfate en tant que produits de combinaison, pour son utilisation en tant que traitement de première intention des candidoses et des candidoses récidivantes, telle que décrite ci-dessus, caractérisée en ce qu'elle est sous la forme d'un comprimé multicouche comprenant :
- au moins une couche à libération immédiate comprenant, par rapport au poids total de l'ensemble des couches à libération immédiate, de 5.10¹⁰ à 1.10¹² UFC de lactobacilles, et 15 à 300 mg de thiosulfate ; et
- au moins une couche à libération prolongée, comprenant, par rapport à l'ensemble des couches à libération prolongée, de 1.10⁷ à 1.10¹⁰ UFC de lactobacilles.

La ou les couches à libération prolongée peuvent également contenir du thiosulfate, avantageusement en une teneur combinée de 60 mg par composition.

La ou les couches à libération prolongée comprennent avantageusement un excipient approprié pour conférer audit comprimé des propriétés de muco-adhésion à la paroi vaginale et de libération prolongée.

Des exemples d'excipients pouvant convenir sont décrits dans Advanced Drug Delivery System Reviews, 57 (2005), 1692-1712. L'excipient peut être choisi parmi le chitosan et ses dérivés, les pectines, les polyéthylènes glycols, l'alginate de sodium, les acides polyacryliques, les dérivés cellulosiques tels que la carboxyméthylcellulose sodique, l'hydroxypropylméthylcellulose ou la cellulose microcristalline, les gommes, les Carbopol^{®} et leurs combinaisons. De préférence, le comprimé selon la présente invention comprend un dérivé cellulosique, en particulier de l'hydroxypropylméthylcellulose (HPMC).

Dans le cadre de l'invention, on préfère utiliser de l'hydroxypropylméthylcellulose de haute viscosité. Ainsi, on utilise avantageusement de l'hydroxypropylméthylcellulose ayant une viscosité dynamique supérieure à 10 000 mPa.s, avantageusement comprise entre 11 000 mPa.s et 21 000 mPa.s, plus avantageusement d'environ 15 000 mPa.s.

La viscosité de cet excipient bien connue est mesurée selon la norme européenne ou américaine pour une solution aqueuse à 2 % (m/v) d'hydroxypropylméthylcellulose à 20°C.

La couche à libération prolongée comprend généralement de 10 à 40 % en poids par rapport au poids total de ladite couche d'un excipient conférant au comprimé ses propriétés de muco-adhésion à la paroi vaginale et de libération prolongée. De préférence, la couche comprend de 10 à 30 % en poids par rapport au poids total de ladite couche dudit excipient, plus avantageusement de 10 à 25 % en poids, encore plus avantageusement de 10 à 20 % en poids, voire elle comprend 10 % en poids dudit excipient. Ledit excipient est avantageusement l'HPMC.

La ou les couches à libération immédiate comprennent avantageusement les composés décrits précédemment pour la composition pharmaceutique, aux teneurs indiquées précédemment.

Le traitement des candidoses, avantageusement des candidoses vulvovaginales et des candidoses vulvovaginales récidivantes, se fera de manière préférée par administration d'une dose jour. La prise pourra éventuellement être renouvelée, par exemple 2, 3, 4 ou 5 jours après la prise de la première dose.

Le traitement des candidoses, avantageusement des candidoses vulvovaginales, et de leur récidive pourra consister en :
- L'administration en première intention de la composition selon l'invention, en une dose, éventuellement renouvelée, par exemple 2, 3, 4 ou 5 jours après la prise de la première dose
- L'administration en prévention d'une récidive d'une composition comprenant des lactobacilles potentialisés par du thiosulfate en une concentration de 10⁷ à 10¹⁰ UFC/g.

### DESCRIPTION DES FIGURES

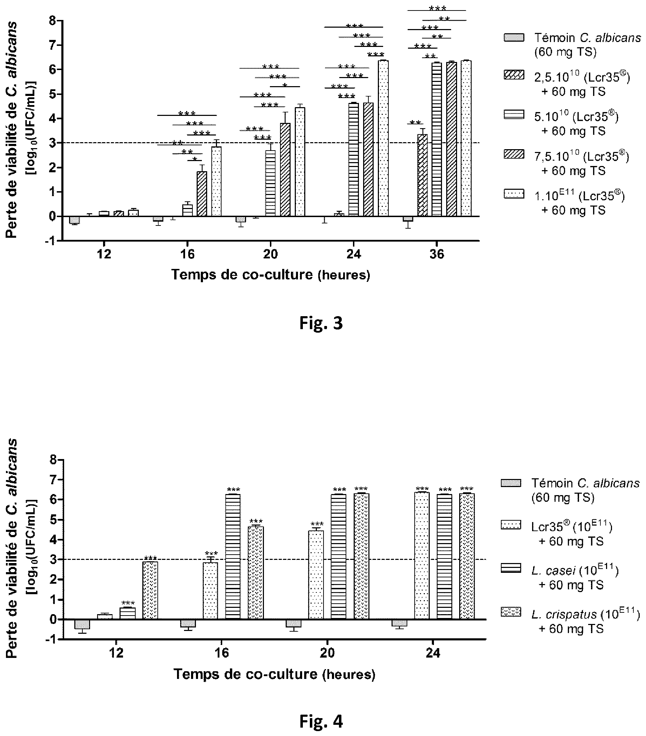

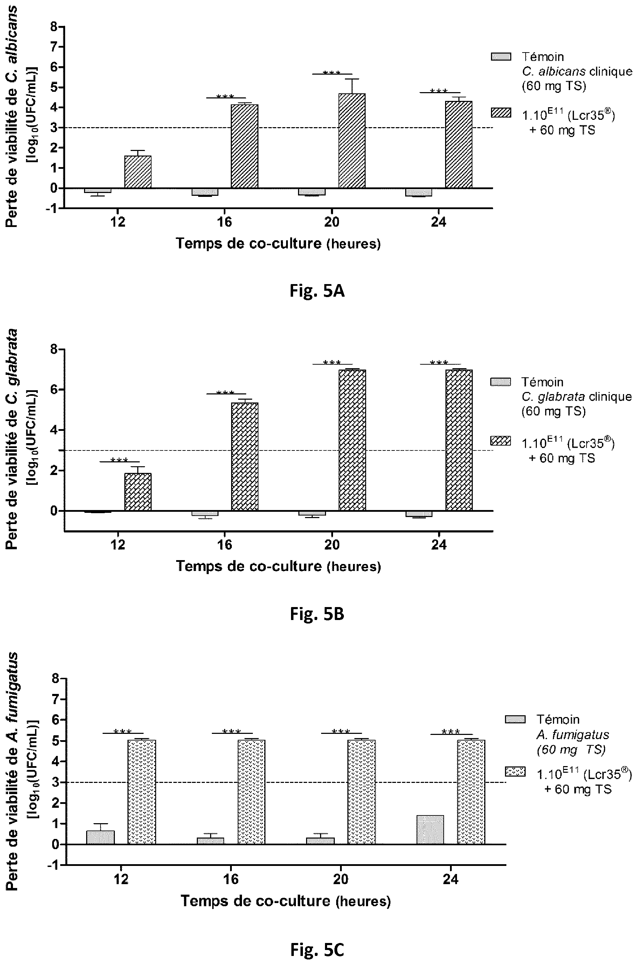

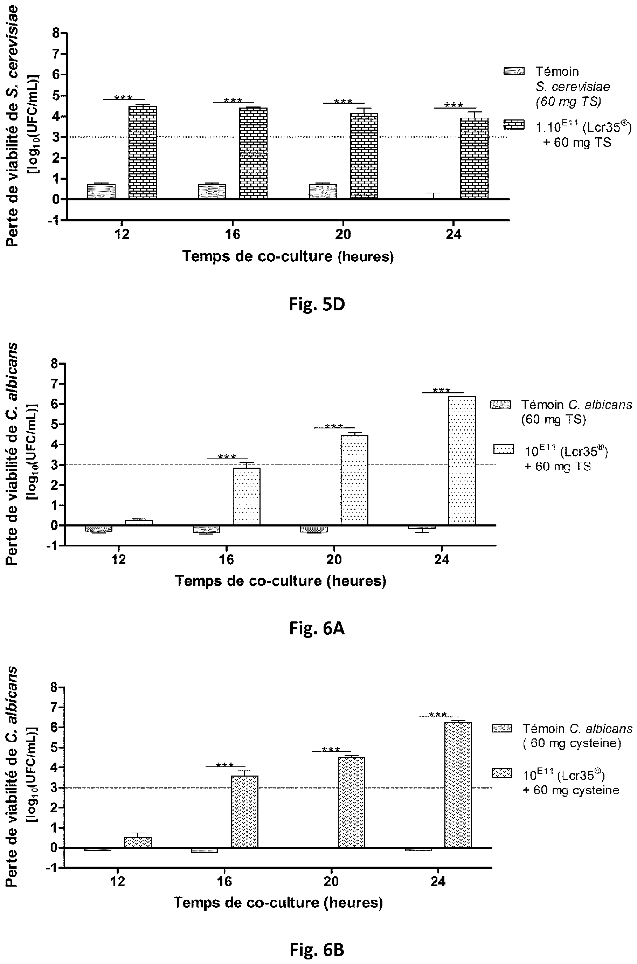

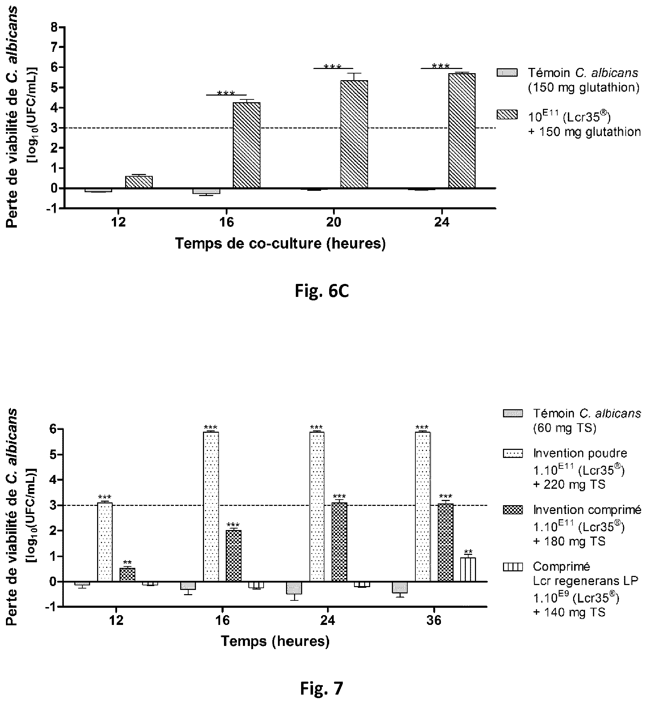

**Figure 1****.** Impact d'une concentration standard (WO2014/009349 vs invention) Perte de la viabilité de *C. albicans* (log₁₀(UFC/ml) en fonction du temps de co-culture (heures)
   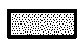 Témoin *C. albicans* (60 mg de thiosulfate) ;
   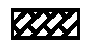 Lcr35^{®} à une concentration de 10⁹ UFC + 30 mg de thiosulfate comme décrite dans WO 2014/009349 ;
   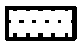 Lcr35^{®} à une concentration de 10¹¹ UFC + 60 mg de thiosulfate ;
**Figure 2****.** Effet de l'association de *L. rhamnosus* Lcr35@ à 10¹¹ UFC + thiosulfate à diverses concentrations
   Perte de la viabilité de *C. albicans* (log₁₀(UFC/ml) en fonction du temps de co-culture (heures)
   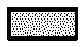 Témoin *C. albicans* (30 mg de thiosulfate) ;
   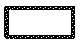 Lcr35^{®} à une concentration de 10¹¹ UFC + 0 mg de thiosulfate ;
   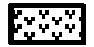 Lcr35^{®} à une concentration de 10¹¹ UFC + 30 mg de thiosulfate ;
   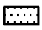 Lcr35^{®} à une concentration de 10¹¹ UFC + 60 mg de thiosulfate ;
   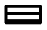 Lcr35^{®} à une concentration de 10¹¹ UFC + 150 mg de thiosulfate ;
   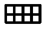 Lcr35^{®} à une concentration de 10¹¹ UFC + 225 mg de thiosulfate ;
   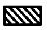 Lcr35^{®} à une concentration de 10¹¹ UFC + 300 mg de thiosulfate
**Figure 3****.** Effet de l'association de *L. rhamnosus* Lcr35^{®} à diverses concentrations + 60 mg de thiosulfate
   Perte de la viabilité de *C. albicans* (log₁₀(UFC/ml) en fonction du temps de co-culture (heures)
   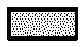 Témoin *C. albicans* (60 mg de thiosulfate) ;
   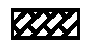 Lcr35^{®} à une concentration de 2,5.10¹⁰ UFC + 60 mg de thiosulfate
   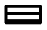 Lcr35^{®} à une concentration de 5.10¹⁰ UFC + 60 mg de thiosulfate ;
   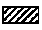 Lcr35^{®} à une concentration de 7,5.10¹⁰ UFC + 60 mg de thiosulfate ;
   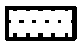 Lcr35^{®} à une concentration de 1.10¹¹ UFC + 60 mg de thiosulfate ;
**Figure 4****.** Effet de l'association de *Lactobacillus* spp. à 10¹¹ UFC + 60 mg de thiosulfate sur *Candida albicans.*
   Perte de la viabilité de *C. albicans* (log₁₀(UFC/ml) en fonction du temps de co-culture (heures)
   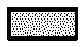 Témoin *C. albicans* (60 mg de thiosulfate) ;
   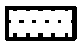 Lcr35^{®} à une concentration de 1.10¹¹ UFC + 60 mg de thiosulfate ;
   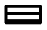 *L. casei* à une concentration de 1.10¹¹ UFC + 60 mg de thiosulfate ;
   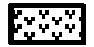 *L. crispatus* à une concentration de 1.10¹¹ UFC + 60 mg de thiosulfate
**Figure 5**. Effet de l'association de *L. rhamnosus* Lcr35^{®} à 10¹¹ UFC + 60 mg de thiosulfate sur *Candida* spp.
   5A : Perte de la viabilité de *Candida albicans* clinique (log₁₀(UFC/ml) en fonction du temps de co-culture (heures)
   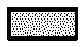 Témoin *C. albicans* clinique (60 mg de thiosulfate) ;
   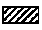 Lcr35^{®} à une concentration de 1.10¹¹ UFC + 60 mg de thiosulfate
   5B : Perte de la viabilité de *Candida glabrata* clinique (log₁₀(UFC/ml) en fonction du temps de co-culture (heures)
   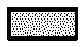 Témoin *C. glabrata* clinique (60 mg de thiosulfate) ;
   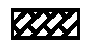 Lcr35^{®} à une concentration de 1.10¹¹ UFC + 60 mg de thiosulfate
   5C : Perte de la viabilité de *Aspergilus fumigatus* (log₁₀(UFC/ml) en fonction du temps de co-culture (heures)
   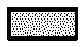 Témoin A. *fumigatus* (60 mg de thiosulfate) ;
   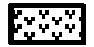 Lcr35^{®} à une concentration de 1.10¹¹ UFC + 60 mg de thiosulfate
   5D : Perte de la viabilité de *Saccharomyces cerevisiae* (log₁₀(UFC/ml) en fonction du temps de co-culture (heures)
   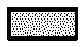 Témoin *S. cerevisiae* (60 mg de thiosulfate) ;
   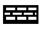 Lcr35^{®} à une concentration de 1.10¹¹ UFC + 60 mg de thiosulfate
**Figure 6****.** Effet de l'association de *L. rhamnosus* Lcr35^{®} à 10¹¹ UFC + de molécules soufrées sur *Candida albicans.*
   Perte de la viabilité de *C. albicans* (log₁₀(UFC/ml) en fonction du temps de co-culture (heures)
   6A : Effet de l'association de Lcr35^{®} à 10¹¹ UFC + 60 mg de thiosulfate
   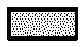 Témoin *C. albicans* (60 mg de thiosulfate) ;
   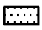 Lcr35^{®} à une concentration de 1.10¹¹ UFC + 60 mg de thiosulfate
   6B : Effet de l'association de Lcr35^{®} à 10¹¹ UFC + 60 mg de cystéine
   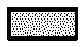 Témoin *C. albicans* (60 mg de Cysteine) ;
   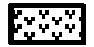 Lcr35^{®} à une concentration de 1.10¹¹ UFC + 60 mg de cystéine
   6C : Effet de l'association de Lcr35^{®} à 10¹¹ UFC + 150 mg de glutathion
   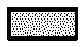 Témoin *C. albicans* (60 mg de glutathion) ;
   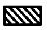 Lcr35^{®} à une concentration de 1.10¹¹ UFC + 150 mg de glutathion
**Figure 7****.** Activité de l'invention sous forme de comprimé
   Perte de la viabilité de *C. albicans* (log₁₀(UFC/ml) en fonction du temps de co-culture (heures) pour une mise en forme de comprimé
   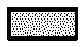 Témoin *C. albicans* (60 mg de thiosulfate) ;
   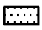 Lcr35^{®} à une concentration de 1.10¹¹ UFC + 220 mg de Thiosulfate(invention sous forme de poudre lyophilisée);
   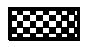 Lcr35^{®} à une concentration de 1.10¹¹ UFC + 180 mg de Thiosulfate (invention sous forme comprimé) ;
   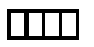 Lcr35^{®} à une concentration de 10⁹ UFC + 140 mg de Thiosulfate comme décrite dans WO 2014/009349, formulée sous forme comprimé.

### EXEMPLES

Effet de l'association de *L. rhamnosus* Lcr35^{®} + thiosulfate sur des pathogènes de différentes espèces de *Candida*

### Matériel et Méthode :

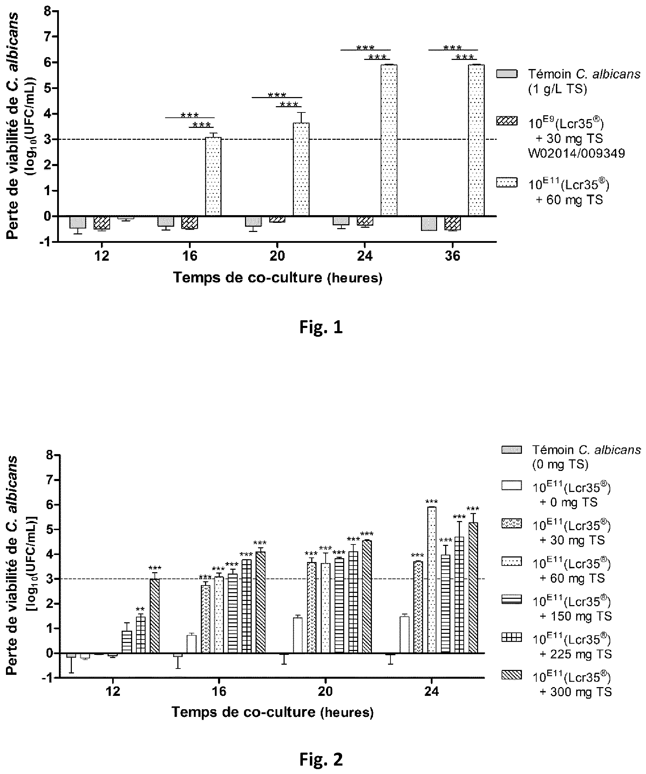

Après une pré-culture dans un bouillon Sabouraud, le pathogène est placé à une numération d'environ 1.10⁸ UFC/ml dans 30 ml de SVF (milieu permettant de simuler le milieu vaginal - pH 4,2) et est mis en contact direct avec les lactobacilles sous forme lyophilisée suite à une étape de fermentation et de lyophilisation + molécule soufrée (thiosulfate ou cystéine) (sans pré-culture afin de s'approcher des conditions d'utilisation *in vivo*). La numération du pathogène est suivi à T0, T12 h, T16 h, T20 h, T24 h par numération sur gélose, et, en plus, à T36h pour les figures 1, 3 et 7. Les résultats sont exprimés en diminution logarithmique entre la numération initiale (T0) et le temps de suivi. L'activité antifongique est établie comme spécifique à partir d'une diminution de 3 log. Un témoin de viabilité du pathogène est réalisé en parallèle dans les mêmes conditions sans présence de composés de l'invention.

On désigne par « formulation A de l'invention WO2014/009349 » une formulation de la souche *L. rhamnosus* Lcr35^{®} telle que préparée par le procédé décrit dans l'exemple 4 de la demande WO2014/009349, c'est-à-dire en présence de 113g/l de thiosulfate de sodium lors de la lyophilisation. Dans les exemples qui suivent, la formulation A comprend 1.10⁹ UFC de *L. rhamnosus* Lcr35^{®} (contre 1.10⁸ UFC pour les exemples de WO2014/009349).

Quatre concentrations en lactobacilles ont été testées : 2,5.10¹⁰ UFC, 5.10¹⁰ UFC, 7,5.10¹⁰ UFC et > 1.10¹¹ UFC soit par ml : 8.10⁸ UFC/ml, 1,6.10⁹ UFC/ml, 2,5.10⁹ UFC/ml et 3,3.10⁹ UFC/ml. Ainsi que la concentration que l'on retrouve dans la formulation A de l'invention WO2014/009349 de 10⁹ UFC soit 3,3. 10⁷ UFC/ml

Différentes concentrations en thiosulfate de sodium dans le milieu de culture de l'essai ont été testées : 0, 1, 2, 5, 7,5, et 10 g/L
Plusieurs pathogènes issus de prélèvements cliniques ont été testés : *Candida albicans, Candida glabrata, Candida tropicalis, aspergilus fumigatus* ainsi qu'une levure de référence *Saccharomyces cerevisiae*

Plusieurs espèces de lactobacilles ont été testées : *L. crispatus, L. casei.* Les résultats sont reportés sur les figures 1 à 6.

### Préparation de comprimés

Le comprimé de la présente invention est un comprimé monocouche formulé pour obtenir une libération contrôlée de la souche *Lactobacillus rhamnosus* Lcr35^{®} à une concentration supérieure ou égale à 1.10¹¹ UFC.g⁻¹ et une quantité de thiosulfate de 180 mg. Le poids du comprimé est d'environ 950mg.

Les comprimés sont préparés grâce à une presse à comprimé de taille industrielle à une force de compression de l'ordre de 20 kN.

### Conclusions :

**Figure 1** : l'invention permet d'avoir une inhibition supérieure à 3 log dès 20 h de co-culture alors que le produit issu de la précédente invention (formulation A décrite dans WO 2014/009349) n'induit aucun effet antifongique sur le pathogène *Candida albicans* ATCC10231.
**Figure 2****.** Dès 1 g/L de thiosulfate (gamme testée de 1 à 10 g/L), l'invention permet d'avoir une inhibition supérieure à 3 log à partir de 20 h de co-culture. A la concentration optimale de 2 g/L, ce niveau d'effet antifongique est atteint dès 16 h de co-culture entre l'invention et le pathogène *Candida albicans* ATCC10231.
**Figure 3** **:** Une activité antifongique de 3 log est obtenue en 24 h à partir d'une concentration en Lcr35^{®} de 5.10¹⁰ UFC (soit 1,6.10⁹ UFC/ml). Pour une concentration de 2,5.10¹⁰ UFC (soit 8.10⁸ UFC/ml), cette activité antifongique est obtenue après 36 h de co-culture entre l'invention et le pathogène *Candida albicans* ATCC10231.
**Figure 4** **:** L'activité antifongique de l'invention est démontrée dès 20 h pour différentes espèces de lactobacilles pour le pathogène *Candida albicans* ATCC10231 en co-culture.
**Figure 5** **:** L'activité antifongique de l'invention est démontrée dès 16 h pour différentes espèces : deux souches cliniques pathogènes : *Candida albicans* et *Candida glabrata ; une souche pathogène : Aspergilus fumigatus et une souche de levure : Saccharomyces cerevisiae.*
**Figure 6** **:** L'activité antifongique de l'invention est démontrée dès 16 h pour différentes sources de molécules soufrées (thiosulfate, cystéine, glutathion) contre le pathogène *Candida albicans* ATCC10231 en co-culture.
**Figure 7** **:** L'activité antifongique est conservée après formulation sous forme de comprimés à libération contrôlée.

### Références bibliographiques

### Publications

1. Sobel JD. Récurrent vulvovaginal candidiasis. A prospective study of the efficacy of maintenance ketoconazole therapy. N Engl J Med 1986; 315: 1455-58.
2. Fischer G. Chronic vulvovaginal candidiasis: what we know and what we have yet to learn. Australas J Dermatol, 2012 Nov;53(4):247-54. doi: 10.1111/j.1440-0960.2011.00860.x. Epub 2012 Sep 24
3. Petrova et al. Lactobacillus species as biomarkers and agents that can promote various aspects of vaginal health Frontiers in Physiology. 2015.
4. Kern et al. Prenventive treatment of vulvovaginal candidosis with vaginal probiotic (Gynophilus® - Lcr Regenerans®) results of the observational study candiflore. La letter du Gynécologue n°370 - mars 2012
5. Yue et al. The dynamic changes of vaginal microecosystem in patients with recurrent vulvovaginal candidiasis; a rétrospective study of 800 patients. Arch Gynecol Obstet DOI 10.1007/s00404-015-3774-2
6. Murina et al. Can Lactobacillus fermentum LF10 and Lactobacillus acidophilus LA02 in a slow-release vaginal product be useful for prévention of recurrent vulvovaginal candidiasis ? A clinical study. J Clin Gastroenterol. Volume 48, sup. 1; November/December 2014
7. Sophie Coudeyras et al. Taxonomic and strain-specific identification of the probiotic strain Lactobacillus rhamnosus 35 within the Lactobacillus casei group, Appl. Environ. Microbiol. doi:10.1128/AEM.02286-07, 2008.

### Brevets et demandes de brevets

US 6,093,394
US 6,468,526
US 7,807,440
US 2010/0151026
WO 84/04675
WO 2000/035465
US 2002/0044926
WO 2006/045475
WO2014/009349
WO 2014/009330

## Revendications

1. Composition pharmaceutique comprenant i) à titre d'actif thérapeutique de 5.10¹⁰ à 1.10¹² UFC de lactobacilles et ii) de 15 à 300 mg de thiosulfate en tant que produits de combinaison, pour son utilisation en tant que traitement de première intention des candidoses et des candidoses récidivantes.

2. Composition pharmaceutique pour son utilisation selon la revendication **1,** pour son utilisation pour inhiber en moins de 24 heures les pathogènes du genre *Candida,* en particulier *Candida albicans, Candida glabrata* ou *Candida tropicalis.*

3. Composition pharmaceutique pour son utilisation selon l'une quelconque des revendications **1** à **2,** pour son utilisation en substitution à un antifongique chimique.

4. Composition pharmaceutique pour son utilisation selon l'une quelconque des revendications **1** à **3, caractérisée en ce qu'**elle comprend de 45 à 120 mg de thiosulfate, avantageusement 45 à 80 mg.

5. Composition pharmaceutique pour son utilisation selon l'une quelconque des revendications **1** à **4, caractérisée en ce que** les lactobacilles sont des *Lactobacillus rhamnosus* et/ou des *Lactobacillus crispatus* et/ou des *Lactobacillus casei.*

6. Composition pharmaceutique pour son utilisation selon l'une quelconque des revendications **1** à **5, caractérisée en ce que** les candidoses sont des candidoses vulvovaginales.

7. Composition pharmaceutique pour son utilisation selon l'une quelconque des revendications **1** à **6, caractérisée en ce qu'**elle est formulée pour être administrée en une prise, une fois par jour.

8. Composition pharmaceutique pour son utilisation selon l'une quelconque des revendications **1** à **7, caractérisée en ce qu'**elle est sous une forme pour administration par voie vaginale, avantageusement sous la forme d'un comprimé ou d'une gélule à libération immédiate.

9. Composition pharmaceutique pour son utilisation selon l'une quelconque des revendications **1** à **8, caractérisée en ce qu'**elle est sous la forme d'un comprimé multicouche comprenant :
- au moins une couche à libération immédiate comprenant, par rapport à l'ensemble des couches à libération immédiate, de 5.10¹⁰ à 1.10¹² UFC de lactobacilles et 15 à 300 mg de thiosulfate ; et
- au moins une couche à libération prolongée, comprenant, par rapport à l'ensemble des couches à libération prolongée, de 1.10⁷ à 1.10¹⁰ UFC de lactobacilles.

10. Composition pharmaceutique pour son utilisation selon la revendication **9, caractérisé en ce que** la couche à libération prolongée comprend de 10 à 40 % en poids par rapport au poids total de ladite couche d'hydroxypropylméthylcellulose.

11. Composition pharmaceutique pour administration par voie vaginale **caractérisée en ce qu'**elle est sous la forme d'un produit de combinaison comprenant i) une première composition pharmaceutique comprenant à titre d'actif thérapeutique de 5.10¹⁰ à 1.10¹² UFC de lactobacilles et ii) une deuxième composition pharmaceutique comprenant de 15 mg à 300 mg de thiosulfate pour son utilisation séparée, simultanée ou décalée dans le temps en tant que traitement de première intention des candidoses et des candidoses récidivantes.

## Patentansprüche

1. Pharmazeutische Zusammensetzung, die i) als therapeutischen Wirkstoff 5.10¹⁰ bis 1.10¹² KbE Laktobazillen und ii) 15 bis 300 mg Thiosulfat als Kombinationsprodukte für ihre Verwendung als Therapie der ersten Wahl von Candidiasis und rezidivierenden Candidiasis umfasst.

2. Pharmazeutische Zusammensetzung für ihre Verwendung nach Anspruch **1** für ihre Verwendung zur Hemmung in weniger als 24 Stunden die Pathogene der Gattung *Candida,* vor allem *Candida albicans, Candida glabrata* oder *Candida tropicalis.*

3. Pharmazeutische Zusammensetzung für ihre Verwendung nach einem der Ansprüche **1** bis **2** für ihre Verwendung als Substitution eines chemischen Antimykotikums.

4. Pharmazeutische Zusammensetzung für ihre Verwendung nach einem der Ansprüche **1** bis **3, dadurch gekennzeichnet, dass** sie 45 bis 120 mg Thiosulfat, in vorteilhafter Weise 45 bis 80 mg, umfasst.

5. Pharmazeutische Zusammensetzung für ihre Verwendung nach einem der Ansprüche **1** bis **4, dadurch gekennzeichnet, dass** die Laktobazillen *Lactobacillus rhamnosus* und/oder *Lactobacillus crispatus* und/oder *Lactobacillus casei* sind.

6. Pharmazeutische Zusammensetzung für ihre Verwendung nach einem der Ansprüche **1** bis **5, dadurch gekennzeichnet, dass** die Candidiasis vulvovaginale Candidiasis sind.

7. Pharmazeutische Zusammensetzung für ihre Verwendung nach einem der Ansprüche **1** bis **6, dadurch gekennzeichnet, dass** sie für eine Verabreichung in einer Einnahme einmal pro Tag formuliert ist.

8. Pharmazeutische Zusammensetzung für ihre Verwendung nach einem der Ansprüche **1** bis **7, dadurch gekennzeichnet, dass** sie in einer Form für eine vaginale Verabreichung ist, in vorteilhafter Weise in Form einer Tablette oder einer Kapsel mit sofortiger Freisetzung.

9. Pharmazeutische Zusammensetzung für ihre Verwendung nach einem der Ansprüche **1** bis **8, dadurch gekennzeichnet, dass** sie in Form einer mehrschichtigen Tablette ist, die umfasst:
- mindestens eine Schicht mit sofortiger Freisetzung, die im Verhältnis zur Gesamtheit der Schichten mit sofortiger Freisetzung 5.10¹⁰ bis 1.10¹² KbE Laktobazillen und ii) 15 bis 300 mg Thiosulfat umfasst; und
- mindestens eine Schicht mit verzögerter Freisetzung, die im Verhältnis zur Gesamtheit der Schichten mit verzögerter Freisetzung 1.10⁷ bis 1.10¹⁰ KbE Laktobazillen umfasst.

10. Pharmazeutische Zusammensetzung für ihre Verwendung nach Anspruch **9, dadurch gekennzeichnet, dass** die Schicht mit verzögerter Freisetzung 10 bis 40 Gew.-% im Verhältnis zum Gewicht der Hydroxypropylmethylcelluloseschicht umfasst.

11. Pharmazeutische Zusammensetzung zur Verabreichung auf vaginalem Weg, **dadurch gekennzeichnet, dass** sie in Form eines Kombinationsprodukts vorliegt, umfassend i) eine erste pharmazeutische Zusammensetzung, die als therapeutischen Wirkstoff 5.10¹⁰ bis 1.10¹² KbE Laktobazillen umfasst und ii) eine zweite pharmazeutische Zusammensetzung, die 15 mg bis 300 mg Thiosulfat umfasst für ihre getrennte, gleichzeitige oder zeitversetzte Verwendung als Therapie der ersten Wahl von Candidiasis und rezidivierenden Candidiasis.

## Claims

1. A pharmaceutical composition comprising i) as therapeutic active agent from 5×10¹⁰ to 1×10¹² CFU of lactobacilli and ii) from 15 to 300 mg of thiosulfate as combination products, for use as a first-line treatment of candidiasis and recurrent candidiasis.

2. The pharmaceutical composition for use according to claim **1,** for use to inhibit in less than 24 hours the pathogens of the genus *Candida,* in particular *Candida albicans, Candida glabrata* or *Candida tropicalis.*

3. The pharmaceutical composition for use according to any one of claims **1** to **2,** for use as a substitute for a chemical antifungal.

4. The pharmaceutical composition for use according to any one of claims **1** to **3, characterized in that** it comprises from 45 to 120 mg of thiosulfate, advantageously from 45 to 80 mg.

5. The pharmaceutical composition for use according to any one of claims **1** to **4, characterized in that** the lactobacilli are *Lactobacillus rhamnosus* and/or *Lactobacillus crispatus* and/or *Lactobacillus casei.*

6. The pharmaceutical composition for use according to any one of claims **1** to **5, characterized in that** the candidiasis are vulvovaginal candidiasis.

7. The pharmaceutical composition for use according to any one of claims **1** to **6, characterized in that** it is formulated to be administered in one dose, once per day.

8. The pharmaceutical composition for use according to any one of claims **1** to **7, characterized in that** it is in a form for vaginal administration, advantageously in the form of an immediate-release tablet or capsule.

9. The pharmaceutical composition for use according to any one of claims **1** to **8, characterized in that** it is in the form of a multilayer tablet comprising:
- at least one immediate-release layer comprising, in relation to all the immediate-release layers, from 5×10¹⁰ to 1×10¹² CFU of lactobacilli and from 15 to 300 mg of thiosulfate; and
- at least one sustained-release layer, comprising, in relation to all the sustained-release layers, from 1×10⁷ to 1×10¹⁰ CFU of lactobacilli.

10. The pharmaceutical composition for use according to claim **9, characterized in that** the sustained-release layer comprises from 10 to 40% by weight of hydroxypropylmethylcellulose in relation to the total weight of said layer.

11. A pharmaceutical composition for vaginal administration **characterized in that** it is in the form of a combination product comprising i) a first pharmaceutical composition comprising as therapeutic active agent from 5×10¹⁰ to 1×10¹² CFU of lactobacilli and ii) a second pharmaceutical composition comprising from 15 mg to 300 mg of thiosulfate for separate, simultaneous or sequential use as a first-line treatment of candidiasis and recurrent candidiasis.
